# EUROPEAN PATENT SPECIFICATION

(11) **EP 3 758 487 B1**
(45) Date of publication and mention of the grant of the patent: **30.08.2023**
(21) Application number: 19758148.1
(22) Date of filing: 26.02.2019
(51) Int. Cl.: A01N 37/44, A01N 25/00, A01P 7/00, A01P 1/00

(54) **METHOD FOR CONTROLLING PATHOGENIC INFECTION OF PLANTS**
METHODE ZUR KONTROLLE PATHOGENER INFEKTIONEN VON PFLANZEN
MÉTHODE DE CONTRÔLE D'INFECTION PATHOGÉNIQUE DE PLANTES

(30) Priority: 26.02.2018 US 201862634992 P; 20.01.2019 US 201962794629 P
(43) Date of publication of application: 06.01.2021
(73) Proprietor: The State of Israel, Ministry of Agriculture & Rural Development, Agricultural Research Organization (ARO) (Volcani Center), 5025001 Beit-Dagan (IL)
(72) Inventor: OREN-SHAMIR, Michal, 7624801 Rehovot (IL); OLIVA, Moran, 7680400 Mazkeret Batia (IL); LEWINSOHN, Efraim, 3657600 Timrat (IL); ALKAN, Noam, 7079500 Moshav Kidron (IL); ELAD, Yigal, 5591333 Ganei-Tikva (IL)
(74) Representative: Boult Wade Tennant LLP
(86) International application number: PCT/IL2019/050218
(87) International publication number: WO 2019/162952

(56) References cited:
- EP-A1- 2 781 157
- WO-A1-2018/042425
- FR-A1- 2 405 650
- I K A Ibrhim , W T Shada , O A I Dawood: "Pathogenicity and control of Meloidogyne incognita on eggplant", Nematologia Mediterranea, vol. 27, no. 1, 10 October 1998 (1998-10-10), pages 31-33, XP055731802,
- Venkataraman G S; Neelakantan S: "Effect of the cellular constituents of the nitrogen-fixing blue-green alga, Cylindrospermum muscicola, on the root growth of rice plants", The Journal of General and Applied Microbiology, vol. 13, no. 1, 31 December 1967 (1967-12-31), pages 53-61, XP055633360, ISSN: 0022-1260, DOI: 10.2323/jgam.13.53
- Dai Yaoren , Wang Jun: "Inhibition of photoperiodic induction of flowering by p- fluorophenylalanine in Japanese morning glory", Chinese Science Bulletin, vol. 33, no. 6, 31 December 1988 (1988-12-31), page 526, XP009523013, CN ISSN: 2095-9273

## Description

### FIELD OF THE INVENTION

The present invention, in some embodiments thereof, relates to a method of controlling a pathogenic infection in a plant.

### BACKGROUND OF THE INVENTION

Damage to plants can be caused by biotic and abiotic stressors. Biotic stressors include arthropods, fungi, viruses, bacteria, moths and nematodes.

Plant disease outbreaks have resulted in catastrophic crop failures that have triggered famines and caused major social change. Plant diseases affect yield quantity and quality. Generally, the best strategy for plant disease control is to use resistant cultivars selected or developed by plant breeders for this purpose. However, the potential for serious crop disease epidemics persists today.

Plants use a wide range of defense mechanisms to avoid infection by pathogens and attack by parasites. These include local induced defense response, formation of local lesions with increased production of reactive oxygen species (ROS), formation of antimicrobial phenolic compounds, deposition of callose and lignin, and induction of pathogenesis related (PR) protein synthesis (Lattanzio et al., 2006). Enhancement in phenol phytoalexins and other aromatic anti-oxidant compounds following biotic stress is the result of the induction of the shikimate pathway synthesizing aromatic amino acids (AAAs) and of downstream specific polyphenol pathways (Pandey et al., 2015; Camañes et al., 2015).

Postharvest losses in plants can be either quantitative or qualitative. Even though emphasis in crop research nowadays is increasing shifting from quantity to production quality, there is still little improvement in the quality of commercially produced plant varieties, hence resulting in high quality losses.

Global regulatory requirements are becoming more and more demanding with respect to the use of pesticides, particularly unmanaged or unnecessary pesticide residues. In addition, the general public would rather consume chemical-free (e.g., less pesticides residue) fruits and vegetables. A particular consequence of this is that there is an increasing need to have more efficient yet safe methods of protecting plants, and products thereof, such as but not limited to fruits and vegetables, from pathogens.

FR2405650A1 describes the application of (L) phenyl alanine to the fight against tracheomycosis in plants.

Ibrahim et al., Nematol. Medit. (1999), vol. 27, no. 1, pages 31-33 describes pathogenicity and control of *Meloidogyne incognita* using thiamine, the amino acids cystic, tryptophane, thyrosine, aspartic acid, and phenylalanine, or the plant regulators gibberlic acidm indolbutyric acid and indol-3-acetic acid, on eggplant.

Venkatamaran and Neelakantan J. Gen. Appl. Microbiol (1967), vol. 13, no. 1, pages 53-61 describes that beside the nitrogenous compounds, other biologically active cell constituents like vitamin B12 and auxins may appreciably contribute to the fertilizing action of the nitrogen-fixing blue-green algae.

Dai and Wang, "Inhibition of photoperiodic induction of flowering by p-fluorophenylalanine in Japanese morning glory", Chinese Science Bulletin, vol. 33, no. 6 (1988) describes that p-fluorophenylalanine inhibited photoperiodic induction of flowering in Japanese morning glory.

EP2781157A1 describes a virus infection inhibitor containing a fermentation by-product selected from an amino acid fermentation by-product and a nucleic acid fermentation by-product that is sprinkled onto a plant body of a plant selected from tobacco, tomato, bell pepper, and chili pepper to control a disease induced by infection of a virus such as Tobamovirus viruses and Cucumovirus viruses.

### SUMMARY OF THE INVENTION

According to the claimed invention, there is provided a method of controlling a pathogenic infection in a plant, comprising contacting the plant with an effective amount of a composition comprising phenylalanine at a concentration of 0.5 to 30 mM, thereby controlling a pathogenic infection in a plant, wherein the pathogenic infection excludes a fungal infection, and wherein the pathogenic infection is an arthropod infection, a bacterium infection, or both.

In some embodiments, the arthropod infection is an insect infection an arachnid infection, or both, and optionally wherein the insect infection is a moth infection.

In some embodiments, the bacterium infection is a *Pseudomonas* infection.

In some embodiments, the concentration of phenylalanine is above 2 mM.

In some embodiments, contacting comprises pre-harvest contacting, post-harvest contacting or a combination thereof.

In some embodiments, contacting is when the plant is at: a post-blossom stage, a blossom stage, a pre-blossom stage, or any combination thereof.

In some embodiments, the plant is a crop.

In some embodiments, phenylalanine is formulated in a composition selected from the group consisting of: a dip, a spray, a seed coating, a concentrate, or any combination thereof.

In some embodiments, contacting is contacting in the vicinity of or onto: a root, a stem, a trunk, a seed, a fruit, a flower, a leave, or any combination thereof.

In some embodiments, contacting is irrigating, drenching, dipping, soaking, injecting, coating, spraying, or any combination thereof.

In some embodiments, contacting is repeated at least twice.

In some embodiments, contacting is pre-infection, post infection, or a combination thereof.

In some embodiments, contacting is in: a storage facility, a greenhouse, an open field, or any combination thereof.

In some embodiments, the moth is *Tuta absoluta.*

In some embodiments, the arachnid is a spider mite (*Tetranychus urticae*)*.*

In some embodiments, the concentration of the phenylalanine is 4-15 mM.

In some embodiments, the composition is used in combination with one or more other agricultural agents, including pesticides, insecticides, acaricides, fungicides, bactericides, and herbicides.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Further embodiments and the full scope of applicability of the present invention will become apparent from the detailed description given hereinafter. The scope of the invention and thus of protection is defined by the appended claims and does not include subject-matter that would not fall within their scope.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

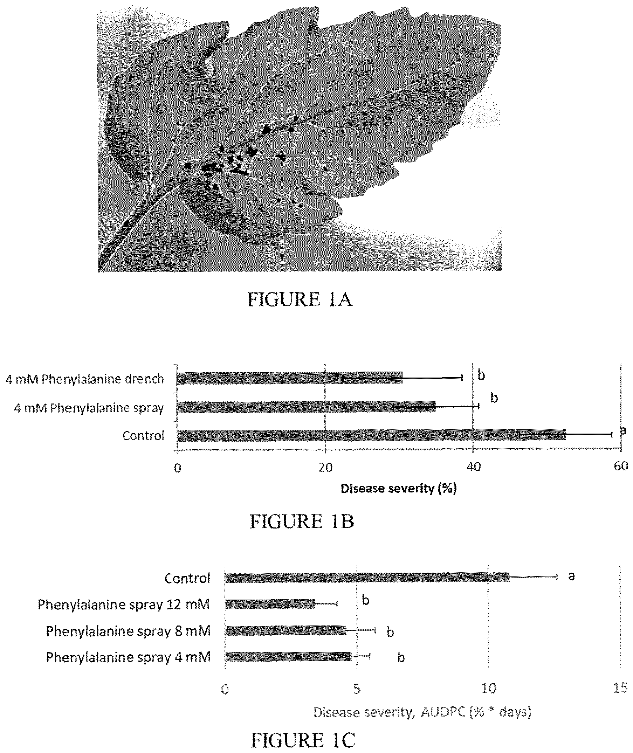

**Figures 1A-1C** are images and graphs showing the effect of 0-12 mM (e.g., 4 mM, and 8 mM) of phenylalanine (Phe) applied by drenching or spraying on the severity of bacterial speck in tomato, 13-14 days post treatment. **Figure 1A****-** an image of tomato bacterial speck on a tomato leaflet caused by the bacterium *Pseudomonas syringae pv. tomato.* **Figure 1B** is a vertical bar graph showing severity of bacterial speck on tomato plants treated with drench or spray of 4 mM solution phenylalanine. Disease was evaluated on a 0-100% severity of symptoms coverage 13 days after treatment. Bars = Standard Errors; Columns followed by a different letter are significantly different (P≤0.05). **Figure 1C** is a vertical bar graph showing severity of bacterial speck on tomato plants treated with spray of 4-12 mM solution phenylalanine. Disease was evaluated on a 0-100 % severity of symptoms coverage and calculation of area under disease progress curve (AUDPC) during 14 days after treatment. Columns followed by a different letter are significantly different (P≤0.05).

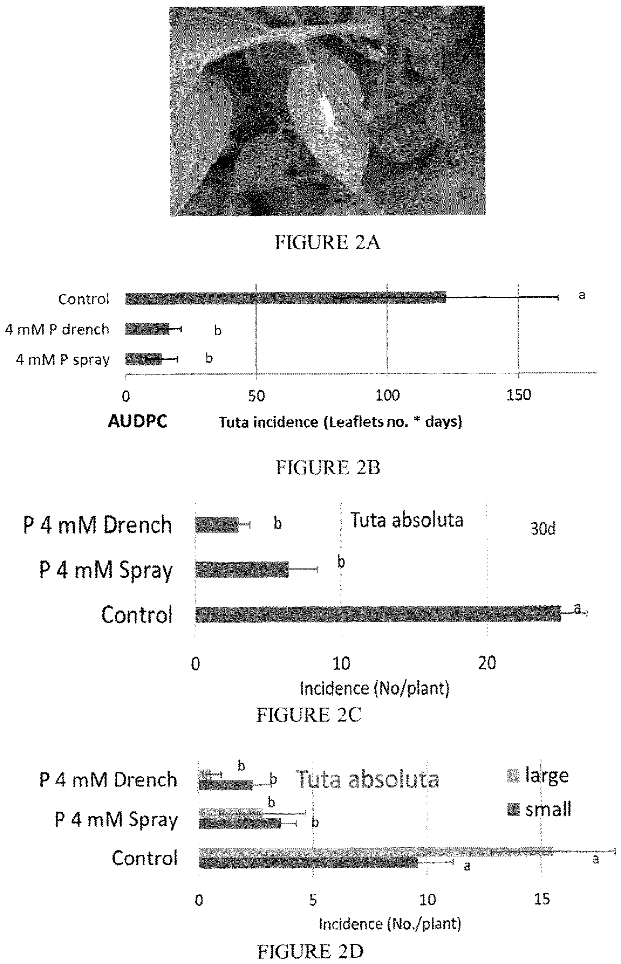

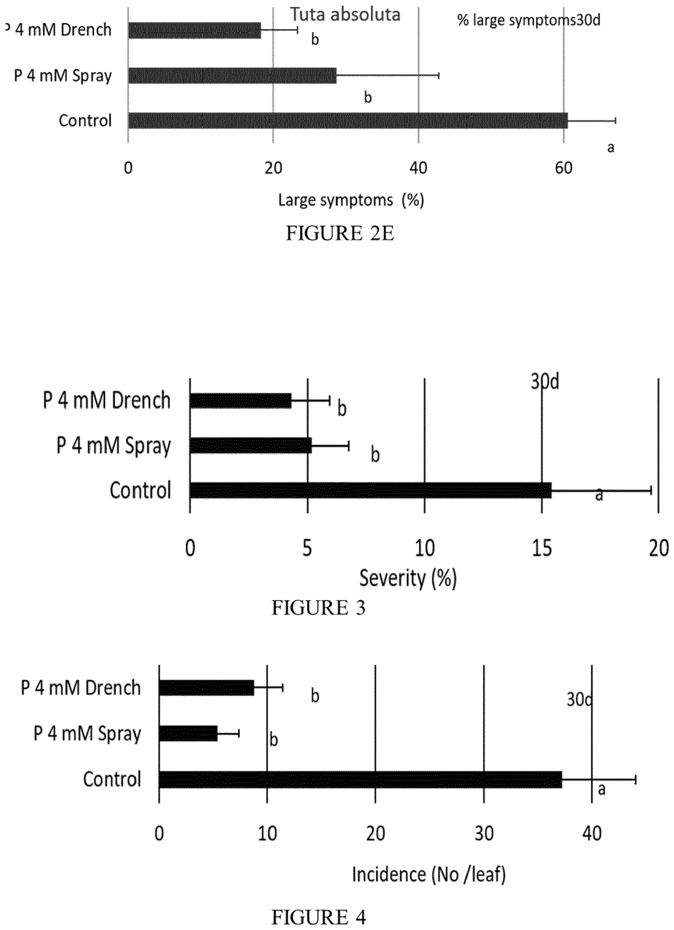

**Figures 2A-2E** are images and bar graphs showing the effect of 4 mM of phenylalanine applied by drenching or spraying on the severity of bacterial speck in tomato, 13 days post treatment. **Figure 2A** - an image showing the damage caused by the tomato leaf miner, the insect moth *Tuta absoluta* on tomato leaflet. **Figure 2B** - a vertical bar graph showing severity of damage of *Tuta absoluta* on tomato leaves treated with drench or spray of 4 mM solution phenylalanine. Disease was evaluated on a 0-100 % severity of symptoms coverage and calculation of area under disease progress curve (AUDPC) 13 days after treatment. Bars = Standard Errors; Columns followed by a different letter are significantly different (P≤0.05). Symptoms incidence per plant was evaluated 30 days after treatment. Bars = Standard Errors; Columns followed by a different letter are significantly different (P≤0.05). **Figure 2C** - a vertical bar graph showing the effect of 4 mM Phe solution on the incidence of tomato leaf miner on tomato plants. The Phe solution was applied by spraying and by drench on the tomato plants at 3 days before and 4 hours before incubation in the greenhouse. Infection was natural. **Figure 2D** - a vertical bar graph showing the effect of 4 mM Phe solution on the incidence of tomato leaf miner on tomato plants. The Phe solution was applied by spraying and by drench on the tomato plants at 3 days before and 4 hours before incubation in the greenhouse. Infection was natural. Symptoms incidences per plant at large size and at smaller size (up to 1 cm) were evaluated 30 days after treatment. Bars = Standard Errors; Columns in each symptom size followed by a different letter are significantly different (P≤0.05). **Figure 2E** - a vertical bar graph showing the effect of 4 mM Phe solution on the rate of tomato leaf miner symptoms on tomato plants. The Phe solution was applied by spraying and by drench on the tomato plants at 3 days before and 4 hours before incubation in the greenhouse. Infection was natural. Symptoms incidences per plant at large size and at smaller size (up to 1 cm) were evaluated 30 days after treatment and the percent of large symptoms was calculated. Bars = Standard Errors; Columns followed by a different letter are significantly different (P≤0.05).

**Figure 3** is a bar graph showing the effect of 4 mM Phe solution on the severity of *Tetranychus urticae* red spider mite on leaves of tomato plants. The Phe solution was applied by spraying and by drench on the tomato plants at 3 days before and 4 hours before incubation in the greenhouse. Symptoms severity was evaluated on a scale of 0-100% severity of symptoms coverage during 30 days after treatment and it is expressed as the area under disease progress curve (AUDPC). Bars = Standard Errors; Columns followed by a different letter are significantly different (*P*≤0.05).

**Figure 4** is a bar graph showing the effect of 4 mM Phe solution on the severity of silver leaf white fly (SLWF) on tomato plants. The Phe solution was applied by spraying and by drench on the tomato plants at 3 days before and 4 hours before incubation in the greenhouse. Incidence of SLWF was evaluated on a representative leaf 30 days after treatment. Bars = Standard Errors; Columns followed by a different letter are significantly different (*P*≤0.05).

### DETAILED DESCRIPTION OF THE INVENTION

The present invention, in some embodiments thereof, relates to a method of controlling pathogen infections and arthropod or pathogen damages in plants.

A plant, according to some embodiments, is any part or tissue of a plant. A plant, according to some embodiments, is a fruit. A plant, according to some embodiments, is a root. A plant, according to some embodiments, is a leaf. A plant, according to some embodiments, is a seed. A plant, according to some embodiments, is a harvested plant.

In one embodiment, a pathogen is any biotic factor, pest or organism that damages and/or infect a plant. In one embodiment, a pathogen is any biotic factor, pest or organism that alters the plant's appearance. In one embodiment, a pathogen is any biotic factor, pest or organism that alters the plant's biochemical content. In one embodiment, a pathogen is any biotic factor that resides on or within the plant.

As used herein, the term "aromatic amino acid (AAA)", refers to phenylalanine (Phe), Tyrosine (Tyr), or Tryptophan (Trp).

In some embodiments, Phe, as utilized herein protects plants from the destructive effects of a pathogen such as but not limited to a bacterium, a moth, an insect or any combination thereof. In some embodiments, Phe inhibits, eliminates, reduces the risk of a pathogen. In some embodiments, Phe protects a plant against pathogenic infection. In some embodiments, Phe treats a plant afflicted with a pathogen. In some embodiments, an activity of Phe as described herein is in a dose dependent manner.

It is therefore contemplated that Phe, can be safely used as an agent against pest and pathogenic stress and/or damage.

Thus, according to an aspect, there is provided a method of controlling a pathogenic infection in a plant, comprising applying to the plant or contacting the plant with an effective amount of a composition comprising Phe.

As used herein, a composition of the invention comprises Phe for use in controlling a pathogenic infection in a plant.

In some embodiments, the composition of the invention comprises Phe.

In one embodiment, a composition comprising Phe is used for inhibiting, ameliorating, treating, preventing and/or controlling a pathogenic infection in the plant, wherein a concentration of Phe, such as phenylalanine or an analog thereof, is 2 mM, 3 mM, 4 mM, 8 mM or above 2 mM. In one embodiment, a concentration of Phe, such as phenylalanine, above 2 mM is used for inhibiting, ameliorating, treating, preventing and/or controlling a pathogenic infection wherein the pathogenic infection is not a fungal infection. In one embodiment, preventing and/or controlling a pathogenic infection includes the elimination of a plant pathogen. In one embodiment, preventing and/or controlling a pathogenic infection includes inhibiting the activity and/or the pathogenic potential of a plant pathogen. In one embodiment, preventing and/or controlling a pathogenic infection includes inhibiting the spread of the pathogen within a plant or to a neighboring plant.

According to an embodiment, provided herein is a method of controlling a pathogenic infection in a plant susceptible thereto such as but not limited to a neighboring plant (a plant in proximity of up to 200 km to an infected plant), the method comprising applying to the plant an effective amount of Phe, such as phenylalanine or an analog thereof, or a composition comprising thereof, for controlling or reducing the risk of pathogenic infection in the plant. In one embodiment, the pathogen is a bacterium. In one embodiment, the pathogen is a *Pseudomonas.* In one embodiment, the pathogen is *Pseudomonas syringae pv tomato.* In one embodiment, the pest is *Tuta absoluta.*

As used herein the term "controlling" refers to preventing, inhibiting, ameliorating a symptom or reducing pathogen infection or arthropod damage or inhibiting the rate, spread and extent of such infection. Curative treatment is also contemplated herein. In one embodiment, controlling comprises inhibiting or stopping the spread of an infection from one plant to another plant. In one embodiment, another plant is a neighboring plant. In one embodiment, inhibiting or stopping the spread of an infection from one plant to another plant comprises treating the infected plant, a neighboring plant or both with Phe, or with a composition comprising thereof, as described herein.

In some embodiments, controlling is treating a plant afflicted with a disease caused by a biotic stressor. In some embodiments, controlling is reducing the risk of infection of uninfected plant or reducing the risk of infection in an uninfected portion of a plant that is infected in other portions. In some embodiments, controlling is reducing the risk of an infection by a stressor as described herein. In some embodiments, controlling is inhibiting or eliminating a plant biotic stressor. In some embodiments, controlling is reducing the risk of contact between a plant and a biotic stressor. In some embodiments, controlling is treating a plant disease caused by a biotic stressor. In some embodiments, a stressor comprises a pathogen. In some embodiments, controlling is ameliorating a pathology associated with infection or resulting from infection as described herein.

According to an embodiment, the bacterium is of the family Pseudomonadaceae. According to an embodiment, the bacterium is Pseudomonas syringae.

According to an embodiment, the controlling is of damage caused by an insect. In one embodiment, controlling is minimizing, inhibiting or reversing a damage.

According to an embodiment, the insect is of the family Gelechiidae. According to an embodiment, the insect is a moth such as but not limited to: Tuta absoluta. According to an embodiment, the controlling is of a pathogenic infection by a nematode.

Following are further examples of plant pathogens and arthropod pests that are contemplated targets for control according to some embodiments of the invention as well as some diseases caused thereby.

**Table 1**

| **Diseases caused by bacteria and controlled by the composition and methods described herein** | | |
|---|---|---|
| **Disease name** | **Causal agent** | **Crops** |
| Deep pitted scab, scab | Streptomyces spp. | Peanut, potato, carrot |
| Bacterial soft rot | Pectobacterium carotovorum subsp. carotovorum | Alfala, Sweet potato, sunflower, maize, potato, onion, carrot, cucurbits, lettuce, eggplant, celery, Brassica spp., tomato, faba bean, pepper, artichoke, garlic, bean, avocado, banana, asclepias, gypsophila, anemone, liatris, pelargonium, carnation, lily, chard, chives |
| Bacterial blight | Pseudomonas syringae pv. syringae | Alfala, Wheat, barley, avocado |
| Bacterial brown spot, leaf spot | Pseudomonas syringae pv. syringae | Pea, cucurbits, pepper |
| Sweet-potato scab, common scab | Streptomyces scabies | Sweet potato, potato |
| Black chaff, bacterial leaf streak | Xanthomonas translucens pv. translucens | Barley |
| Bacterial leaf spot | Pseudomonas syringae | Clover |
| Bacterial fruit blotch | Acidovorax avena subsp. citrulli | Cucurbits |
| Bacterial leaf spot and head rot | Xanthomonas campestris | Lettuce |
| Bacterial leaf spots of Umbelliferae | Xanthomonas campestris pv. carotae | Carrot, celery |
| Bacterial spot | Xanthomonas campestris pv. vesicatoria | Tomato, pepper |
| Bacterial canker | Clavibacter michiganensis subsp. michiganensis | Tomato, pepper |
| Pith necrosis | Pseudomonas corrugata | Tomato |
| Bacterial speck | Pseudomonas syringae pv. tomato | Tomato |
| Stewart's wilt of corn | Erwinia stewartii | Maize |
| Fireblight | Erwinia amylovora | Apple, crabapples, loquat, pear |
| Bacterial crown gall | Rhizobium radiobacter | Sweet potato, rose, grapevine, carrot, beet, tomato, pepper, avocado, pear, peach, cherry, olive, apricot, plum, loquat, almond, fig, apple, eucalyptus, aster, anemone, buttercup, carnation, chard |
| Citrus canker | Xanthomonas axonopodis | Citrus |
| Citrus greening | Candidatus Liberibacter | Citrus |
| Bacterial blight | Xanthomonas oryzae pv. oryzae | Rice |
| Bacterial leaf streak | Xanthomonas oryzae pv. oryzicola | Rice |
| Foot rot | Erwinia chrysanthemi. | Rice |
| Grain rot | Burkholderia glumae | Rice |

**Table 2**

| **Insect pests** | | |
|---|---|---|
| **Common name** | **Scientific species** | **Crops** |
| Tomato leafminer | Tuta absoluta | Tomato, potato, eggplant, pepino, pepper, tobacco |
| Olive fly | Bactrocera oleae | Olive |
| Greater date moth | Aphomia (Arenipses) sabella | Date palm |
| Sweet potato whitefly | Bemisia tabaci | Tomato, peppers, squash, cucumber, beans, eggplant, watermelon, cabbage, potato, peanut, soybean, cotton many ornamental host plants: poinsettia, hibiscus, chrysanthemum and any other crops |
| Corn aphid | Rhopalosiphum maidis | Sorghum, cereals |
| Cowpea aphid | Aphis craccivora | Bean, cowpea |
| Green peach aphid | Myzus persicae | Peach, cotton, rapeseed |
| Oat aphid | Rhopalosiphum padi | Barley, wheat, oats |
| Pea aphid | Acyrthosiphon pisum | Chickpea, pea, lucerne |
| Rice root aphid | Rhopalosiphum rufiabdominalis | Rice |
| Soybean aphid | Aphis glycine | Soybean, Glycine spp. |
| Spotted alfalfa aphid | Therioaphis trifolii | Lucerne |
| Turnip aphid | Lipaphis erysimi | Rapeseed |
| Wheat aphid | Rhopalosiphum padi | Barley, wheat and oats |
| **Common armyworm** | Leucania convecta | barley, oats, wheat, native pasture grasses and perennial grass seed crops |
| **Northern armyworm** | Leucania separata | sorghum, maize, barley, wheat and rice |
| Large brown bean bug | Riptortus serripes | Soybeans, pulses, cotton and many horticultural crops |
| Small brown bean bug | Melanacanthus scutellaris | Pulses |
| Brown flea beetle | Chaetocnema sp. | Cotton |
| Brown mirid | Creontiades pacificus | Cotton, lucerne, mungbeans, navy beans, peanut and soybeans |
| Cabbage moth | Plutella xylostella | Brassica plants |
| Budworm | Helicoverpa punctigera | cotton, chickpea, sunflower, soybean, mungbean, navy bean, lucerne, canola, peanut, faba bean, safflower, linseed |
| Cotton bollworm, corn earworm | Helicoverpa armigera | , wheat and barley |
| Crop mirid | Sidnia kinbergi | Cotton, lucerne, mungbeans, navy beans, peanut, soybeans |
| Diamondback moth | Plutella xylostella | Rapeseed |
| False wireworm | Pterohelaeus and Gonocephalum spp | Maize |
| Greenhouse whitefly | Trialeurodes vaporariorum | Cotton, sunflower, soybean and navy bean |
| Cotton Leafhopper | Amrasca terraereginae | Cotton |
| Lesser armyworm | Spodoptera exigua | Cotton |
| Onion thrips | Thrips tabaci | Cotton, navy bean, mungbean, cereals |
| Soybean leafminer | Porphyrosela aglaozona | Soybean |
| Soybean looper | Thysanoplusia orichalcea | Soybean |
| Soybean moth | Aproaerema simplexella | Soybean |
| Tobacco Thrips | Thrips tabaci | Cotton, bean, mungbean, cereals |
| Western flower thrips | Frankliniella orientalis | Cotton, navy bean, mungbean, sunflower, canola, peanut, other crops |

**Table 3**

| **Arachnid pests** | | |
|---|---|---|
| **Common name** | **Scientific species** | **Crops** |
| Broad mite | Polyphagotarsonemus latus | Pepper, sweet basil, apple, avocado, cantaloupe, castor, chili, citrus, coffee, cotton, eggplant, grapes, guava, jute, mango, papaya, passion fruit, pear, potato, sesame, beans, tea, tomato, African violet, ageratum, azalea, begonia, chrysanthemum, cyclamen, dahlia, gerbera, gloxinia, ivy, jasmine, impatiens, lantana, marigold, peperomia, pittosporum, snapdragon, verbena, zinni |
| Mite, Bean spider | Tetranychus ludeni | Cotton, bean, soybean |
| Two-spotted Mite | Tetranychus urticae | horticultural and field crops, including maize, cotton, soybean, canola, lucerne, peanut, mungbean, beans |

A plant, in some embodiments, is a crop. In another embodiment, a plant is a fruit or a vegetable. In another embodiment, a plant refers either to the harvested parts or to the harvest in a more refined state (husked, shelled, etc.). In another embodiment, a plant is a cultivated plant. In another embodiment, a plant is a fodder crop. In another embodiment, a plant includes horticulture, floriculture and industrial crops.

In another embodiment, a plant belongs to the superfamily Viridiplantae. In another embodiment, a plant is a monocotyledonous. In another embodiment, a plant is a dicotyledonous plant. In another embodiment, a plant is: Acacia spp., Acer spp., Actinidia spp., Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chacoomeles spp., Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Dibeteropogon amplectens, Dioclea spp, Dolichos spp., Dorycnium rectum, Echinochloa pyramidalis, Ehraffia spp., Eleusine coracana, Eragrestis spp., Erythrina spp., Eucalypfus spp., Euclea schimperi, Eulalia vi/losa, Pagopyrum spp., Feijoa sellowlana, Fragaria spp., Flemingia spp, Freycinetia banksli, Geranium thunbergii, GinAgo biloba, Glycine javanica, Gliricidia spp, Gossypium hirsutum, Grevillea spp., Guibourtia coleosperma, Hedysarum spp., Hemaffhia altissima, Heteropogon contoffus, Hordeum vulgare, Hyparrhenia rufa, Hypericum erectum, Hypeffhelia dissolute, Indigo incamata, Iris spp., Leptarrhena pyrolifolia, Lespediza spp., Lettuca spp., Leucaena leucocephala, Loudetia simplex, Lotonus bainesli, Lotus spp., Macrotyloma axillare, Malus spp., Manihot esculenta, Medicago saliva, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativam, Podocarpus totara, Pogonarthria fleckii, Pogonaffhria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys vefficillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp., Vitis vinifera, Watsonia pyramidata, Zantedeschia aethiopica, Zea mays, amaranth, artichoke, asparagus, broccoli, Brussels sprouts, cabbage, canola, carrot, cauliflower, celery, collard greens, flax, kale, lentil, oilseed rape, okra, onion, potato, rice, soybean, straw, sugar beet, sugar cane, sunflower, tomato, squash tea, maize, wheat, barley, rye, oat, peanut, pea, lentil and alfalfa, cotton, rapeseed, canola, pepper, sunflower, tobacco, eggplant, eucalyptus, a tree, an ornamental plant, a perennial grass and a forage crop. Alternatively, algae and other non-Viridiplantae can be used for the methods of the present invention.

According to an embodiment, the plant is not an ornamental plant (e.g., African daisy, bellflower, butterfly flower, sunflower, sapphire flower, safflower, rose, poinsettia, monkey-flower, geranium, fuchsia, carnation, dahlia, Araceae, Acanthaceae, Agavaceae, Araliaceae, Asclepiadaceae, Gesneriaceae, Ficus, Polypodiaceae, Vitaceae, rhododendron).

According to some embodiments of the invention, the plant used by the method of the invention is a crop plant such as rice, maize, wheat, barley, peanut, potato, sesame, olive tree, palm oil, banana, soybean, sunflower, canola, sugarcane, alfalfa, millet, leguminosae (bean, pea), flax, lupinus, rapeseed, tobacco, poplar and cotton.

According to some embodiments of the invention the plant is a dicotyledonous plant. According to some embodiments of the invention the plant is a monocotyledonous plant. According to an embodiment, the plant is susceptible to infection by (e.g., any of the above) virus, bacterium, arthropod, insect or nematode. According to an embodiment, the plant is infected by or susceptible for infection or damage by (e.g., any of the above) virus, bacterium, insect, arachnid, or nematode. According to an embodiment, the plant is grown in a habitat infested by (e.g., any of the above) virus, bacteria, insect, nematode, or arachnid (e.g., spider mite).

According to an embodiment, the plant is a cultivated fruit plant. According to an embodiment, the cultivated fruit plant, refers to a plant which fruits are of an economic value. According to an embodiment, the cultivated fruit plant is selected from the group consisting of strawberries, grapes, apples, blueberries, cherries. According to an embodiment, the cultivated fruit plant is not strawberry, peach, apple, orange, lemon, lime, plum, cherry, raspberry, blackberry, tomato, pepper, melon, cucumber, squash, watermelon (when applied to grains or fruits). According to an embodiment, the plant is not a snapdragon, petunia or lisianthus.

As used herein the term, "Phenylalanine" or "Phe" refers to the α-amino acid with the formula C₉H₁₁NO₂. It can be viewed as a benzyl group substituted for the methyl group of alanine, or a phenyl group in place of a terminal hydrogen of alanine. This essential amino acid is classified as neutral, and nonpolar because of the inert and hydrophobic nature of the benzyl side chain. The L-isomer is used to biochemically form proteins, coded for by DNA. The codons for L-phenylalanine are UUC and UUU. Phenylalanine is a precursor for tyrosine; the monoamine neurotransmitters dopamine, norepinephrine (noradrenaline), and epinephrine (adrenaline); and the skin pigment melanin.

According to an embodiment the Phe is at a concentration of 0.5-30 mM, 10-30 mM, 5-30 mM, 1-30 mM, 10-20 mM, 0.5-20 mM, 5-20 mM, 1-20 mM, 1-15 mM, or 15 to 30 mM.

According to an embodiment Phe is administered or applied (or contacted) at a concentration of above 2 mM, e.g., above 3 mM, above 4 mM, above 5 mM, above 10 mM, above 15 mM, above 20 mM, above 25 mM, or above 30 mM.

According to an embodiment Phe is administered at a concentration of 0.8-1.5 mM, 1-2.5 mM, 2.2-8 mM, 2.5-10 mM, 3-10 mM, 2.2-8 mM, 4-12 mM, 4-15 mM, 10-30 mM, 5-30 mM, 2.5-30 mM, 10-20 mM, 2.5-20 mM, 5-20 mM, or 15 to 30 mM. According to one embodiment, at least 2 mM is more than 2 mM.

According to an embodiment, Phe is administered at a concentration of 4-12 mM.

According to an embodiment, Phe is administered at a concentration that does not cause sedimentation on the plant. According to an embodiment, Phe is administered at a concentration that does not cause Phe sedimentation on the plant. According to an embodiment, Phe is administered at a concentration lower than 50 mM.

In one embodiment, the term "treated", "prevented", "administered", "applied", "controlled" and "contacted" are interchangeable or synonymous.

As used herein "plant" refers to whole plants, a plant tissue, a plant organ, a fruit, a vegetable, an eatable portion of a plant, a grafted plant including seeds, shoots, stems, roots (including tubers), rootstock, scion, and plant cells, tissues, fruit, flower and organs. The plant may be in any form including cuttings and harvested material (e.g., fruit).

The Phe can be applied to plants by spraying, dusting, coating, soaking, irrigation, drenching or otherwise treating them with the active ingredients or alternatively, by treating with the active ingredients the plant seeds, the soil around the plant, or the soil, rice pads or the water for hydroponic culture where the seeds are to be sown. The application may be affected either before or after the plant is infected with a pathogen.

According to an embodiment, the regimen is performed such as to control the spread of a pathogen and/or eliminate a pathogen and/or eliminate/reduce/minimize any damage that can be caused by the pathogen.

According to an embodiment, applying comprises pre-harvest applying. According to an embodiment, applying comprises post-harvest applying. According to an embodiment, applying comprises pre-harvest applying and not post-harvest applying. According to an embodiment, applying is multiple administrations of Phe. According to an embodiment, applying comprises post-harvest applying and not pre-harvest applying. According to an embodiment, the plant is at a post-blossom stage. According to an embodiment, the plant is at a blossom stage. According to an embodiment, plant is at a pre-blossom stage. According to an embodiment, applying includes daily applying, weekly applying, bi-weekly applying, monthly applying, bi-monthly applying, seasonally applying etc.

In one embodiment, postharvest treatment with at least 2.5 mM phenylalanine reduced the postharvest decay severity (caused by fungi or bacteria). In one embodiment, postharvest treatment with at least 4 mM phenylalanine reduced the postharvest decay severity (caused by fungi or bacteria). In one embodiment, postharvest treatment comprises postharvest treatment of a fruit. In one embodiment, postharvest treatment comprises postharvest treatment of a crop. In one embodiment, postharvest treatment comprises postharvest treatment of a vegetable. In one embodiment, postharvest treatment comprises postharvest treatment of a plant. In one embodiment, postharvest treatment comprises postharvest treatment of a flower.

When indicated a specific stage, the application can be confined only to this stage or to the recited stage and additionally to another stage. For instance, when indicated applying at blossom, applying can be performed at blossom or blossom+ post-blossom (i.e., fruit), or pre-blossom and blossom or pre-blossom and blossom and post blossom. According to an embodiment, applying is post-emergence (of the infection by a pathogen). According to an embodiment, said phenylalanine or analog is formulated in a composition selected from the group consisting of a dip, a spray or a concentrate. According to an embodiment, applying is in the vicinity of or onto the roots, stems, trunk, seed, fruits or leaves of the plant. According to an embodiment, applying is by irrigation, drenching, dipping, soaking, injection, coating or spraying. According to an embodiment, applying is in an open field. According to an embodiment, applying is in a greenhouse. According to an embodiment, applying is in a storage facility (e.g., dark room, refrigerator). According to an embodiment, applying is applying once. According to an embodiment, applying is applying at least twice at any regimen or duration as necessary and/or as described herein.

According to an embodiment, applying comprises repeated application (2 or more applications e.g., every week, seasonal, bi-weekly, bi-monthly etc.). Repeated applications are especially envisaged for field/greenhouse treatments.

According to an embodiment, repeated application comprises weekly, daily, monthly, or bi-monthly administration during blossom, post-blossom, pre-blossom, or any combination thereof. For example, suggested regimen may include but is not limited to, spraying plants in open fields and green house, adding to irrigation of plants grown in the open field, green house and in pots, dipping the whole foliage branch in the solution post-harvest, adding to vase of cut flowers before and/or after harvest and possibly before shipment.

According to an embodiment, the active ingredient (Phe) is formulated into a composition where it is mixed with other active ingredients (e.g., fungicides) and/or an agriculturally acceptable carrier.

According to an embodiment such a composition of the invention is shelf stable. The term "shelf stable" refers to a composition of the invention that maintains its activity throughout a given storage period at the recommended conditions (e.g., temperature) and optionally does not separate out into separate phases or develop any offensive odors.

As used herein the term "agriculturally acceptable carrier" refers to a material that facilitates application of a composition of the invention to the intended target, which may be for example a plant, a plant material, compost, earth, surroundings or equipment, or that facilitates storage, transport or handling. Carriers used in compositions for application to plants and plant material are preferably non-phytotoxic or only mildly phytotoxic. A suitable carrier may be a solid, liquid or gas depending on the desired formulation. In one embodiment the carriers include polar liquid carriers such as water, mineral oils and vegetable oils. In one embodiment the carrier enhances the stability of the active ingredient as described herein.

Examples of liquid carriers include but are not limited to water; alcohols, particularly butanol or glycol, as well as their ethers or esters, particularly methylglycol acetate; ketones, particularly acetone, cyclohexanone, methylethyl ketone, methylisobutylketone, or isophorone; petroleum fractions such as paraffinic or aromatic hydrocarbons, particularly xylenes or alkyl naphthalenes; mineral or vegetable oils; aliphatic chlorinated hydrocarbons, particularly trichloroethane or methylene chloride; aromatic chlorinated hydrocarbons, particularly chlorobenzenes; water-soluble or strongly polar solvents such as dimethylformamide, dimethyl sulfoxide, or N-methylpyrrolidone; liquefied gases; or the like or a mixture thereof.

Examples of solid carriers include but are not limited to fillers such as kaolin, bentonite, dolomite, calcium carbonate, talc, powdered magnesia, Fuller's earth, gypsum, diatomaceous earth and China clay. A carrier which provides for slow or delayed release of a compound (Phe) of the invention may also be included in a composition of the invention (especially for the short life cycle pathogens).

In another embodiment, a composition (or active ingredient thereof - Phe) of the invention is applied in an amount capable of inhibiting germination of bacterial spores or bacterial spreading. According to an embodiment, the composition (or active ingredient thereof - Phe) of the invention is applied in an amount capable of reducing the standard concentration advised by a regulatory agency (e.g., FDA, USDA) of commonly used agrotech formulations.

According to an embodiment, the composition (or active ingredient thereof - Phe) of the invention is applied in an amount able to reduce symptoms, spread and/or severity of infection dependent on the pathogen.

As used herein "increasing" or "decreasing" or "reducing" refers to about +/- at least 10 %, 20 %, 30 %, 40 %, 50 %, 60 %, 70 %, 80 %, 90 % or more compared to a control plant in the absence of Phe or analog under identical assay conditions.

Applying may be directly to the plant or to a surface in sufficient vicinity to the plant to control the pathogen infection of the plant. In one embodiment, vicinity is within a distance of 1 meter. In one embodiment, vicinity is within a distance of 0.7 meter. In one embodiment, vicinity is within a distance of 0.5 meter. In one embodiment, vicinity is within a distance of 0.2 meter.

Thus, applying can be to any target surface of a plant or a plant organ to which a compound or composition of the invention may be applied, for example to a plant, plant material including roots, bulbs, fruit, tubers, corms, leaves, flowers, seeds, stems, callus tissue, nuts, grains, fruit, cuttings, root stock, scions, harvested crops including roots, bulbs, tubers, corms, leaves, flowers, seeds, stems, callus tissue, nuts, grains, fruit, cuttings, root stock, scions, or any surface that may contact harvested crops including harvesting equipment, packaging equipment and packaging material.

For surfaces such as harvesting equipment, packaging equipment and packaging material, the compound or composition of the invention is applied before use of the harvesting equipment, packaging equipment or packaging material.

According to an embodiment, the compound or composition of the invention is formulated as a dip, a powder, a spray or a concentrate. According to an embodiment, the formulation comprises a surfactant. According to an embodiment, the surfactant is a cationic surfactant, e.g., benzalkonium chloride, cetylpyridinium chloride. According to an embodiment, the surfactant is an anionic surfactant, e.g., alkyl sulphates, alkyl ethoxylate sulphates. According to an embodiment, the surfactant is a non-ionic surfactant, e.g., Alkyl polyglycoside, Triton X-100, Polyoxyethylene (20) sorbitan monooleate (Tween-80), Silwett L-77. According to an embodiment, the surfactant is Tween-80 or Silwett L-77.

According to an embodiment, the concentration of the surfactant is at least 0.1 %. In one embodiment, a composition of the invention may further comprise at least one additional agricultural agent. In an alternative embodiment a composition of the invention may be delivered separately, simultaneously or sequentially with at least one additional agricultural agent.

In one embodiment, a composition of the invention may further comprise at least one additional agricultural agent e.g., fungicide, antibiotic, nematocide and/or insecticide. In an alternative embodiment a composition of the invention may be delivered separately, simultaneously or sequentially with at least one additional agricultural agent.

When formulating a composition of the invention containing an additional agricultural agent or planning delivery of a composition of the invention separately, simultaneously or sequentially with an additional agricultural agent, it may be desirable to assess the degree of phytotoxicity resulting from application of the compositions to plant material over time. This may be assessed according to the methodology well known in the art.

In some embodiments, Phe can be in a composition with a coating agent, such as but not limited to, a polysaccharide.

Assessment of a composition of the invention or a composition of the invention including or delivered with an additional agricultural agent may include assessment of: (1) Degree of control of the pathogen without stimulating growth of undesirable non-target microbes or harming beneficial organisms. (2) Durability of control. (3) Degree of phytotoxicity and effects on plant development when used repeatedly throughout a portion or the entirety of a growing season. (4) Compatibility with other control products used in the industry.

As described above, the compositions of the present invention may be used alone or in combination with one or more other agricultural agents, including pesticides, insecticides, acaricides, fungicides, bactericides, herbicides, antibiotics, antimicrobials, nematocides, rodenticides, entomopathogens, pheromones, attractants, plant growth regulators, plant hormones, insect growth regulators, chemosterilants, microbial pest control agents, repellents, viruses, phagostimulants, plant nutrients, plant fertilizers and biological control agents. When used in combination with other agricultural agents the administration of the two agents may be separate, simultaneous or sequential. Specific examples of these agricultural agents are known to those skilled in the art, and many are readily commercially available.

Examples of plant nutrients include but are not limited to nitrogen, magnesium, calcium, boron, potassium, copper, iron, phosphorus, manganese, molybdenum, cobalt, boron, copper, silicon, selenium, nickel, aluminum, chromium and zinc. Examples of antibiotics include but are not limited to oxytetracycline and streptomycin. Examples of fungicides include but are not limited to the following classes of fungicides: carboxamides, benzimidazoles, triazoles, hydroxypyridines, dicarboxamides, phenylamides, thiadiazoles, carbamates, cyano-oximes, cinnamic acid derivatives, morpholines, imidazoles, beta-methoxy acrylates and pyridines/pyrimidines. Further examples of fungicides include but are not limited to natural fungicides, organic fungicides, Sulphur-based fungicides, copper/calcium fungicides and elicitors of plant host defenses.

Examples of natural fungicides include but are not limited to whole milk, whey, fatty acids or esterified fatty acids. Examples of organic fungicides include but are not limited to any fungicide which passes an organic certification standard such as biocontrol agents, natural products, elicitors (some of may also be classed as natural products), and Sulphur and copper fungicides (limited to restricted use).

An example of a Sulphur-based fungicide is Kumulus^{™} DF (BASF, Germany). An example of a copper fungicide is Kocide.RTM. 2000 DF (Griffin Corporation, USA). Examples of elicitors include but are not limited to chitosan, Bion^{™}, BABA (DL-3-amino-n-butanoic acid, beta -aminobutyric acid) and Milsana^{™} (Western Farm Service, Inc., USA).

In some embodiments non-organic fungicides may be employed. Examples of non-organic fungicides include but are not limited to Bravo^{™} (for control of powdery mildew (PM) on cucurbits); Supershield^{™} (Yates, NZ); Topas.RTM. 200EW (for control of PM on grapes and cucurbits); Flint^{™} (for control of PM on apples and cucurbits); Amistar.RTM. WG (for control of rust and PM on cereals); and Captan^{™}, Dithane^{™}, Euparen^{™}, Rovral^{™}, Scala^{™}, Shirlan^{™}, Switch^{™} and Teldor^{™}.

Examples of pesticides include but are not limited to azoxystrobin, bitertanol, carboxin, Cu₂O, cymoxanil, cyproconazole, cyprodinil, dichlofluamid, difenoconazole, diniconazole, epoxiconazole, fenpiclonil, fludioxonil, fluquiconazole, flusilazole, flutriafol, furalaxyl, guazatin, hexaconazole, hymexazol, imazalil, imibenconazole, ipconazole, kresoxim-methyl, mancozeb, metalaxyl, R-metalaxyl, metconazole, oxadixyl, pefurazoate, penconazole, pencycuron, prochloraz, propiconazole, pyroquilon, SSF-109, spiroxamine, tebuconazole, thiabendazole, tolifluamid, triazoxide, triadimefon, triadimenol, triflumizole, triticonazole and uniconazole.

Efficacy of compositions of the invention may also be confirmed using field trial assay systems. For example, confirmation of the ability of compositions of the invention to prevent pathogen growth may be obtained by applying a compound or composition of the invention to plant material and then inoculating with a target organism. Efficacy is confirmed by the absence of growth or less growth of the target organism than an untreated control. According to an embodiment the agricultural composition may comprise phenylalanine or an analog thereof and a surfactant (as described herein) for controlling a pathogen infection in a plant, in an open-field and/or a greenhouse.

According to an embodiment the agricultural composition may comprise phenylalanine and tyrosine for controlling a pathogen infection in a plant.

As used herein the term "about" refers to ± 10 %.

The terms "comprises", "comprising", "includes", "including", "having" and their conjugates mean "including but not limited to".

The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure. As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts. As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

### EXAMPLES

Reference is now made to the following examples, which together with the above descriptions illustrate some embodiments of the invention in a non-limiting fashion.

### EXAMPLE 1

### Tomato bacterial speck

### Background

Causal agent: *Pseudomonas syringae pv. tomato* (Okabe).

Symptoms of bacterial speck are small spots that appear on the leaves of the tomato plant. The spots are small and brown in the center, surrounded by a yellow ring. In severe cases the spots may overlap and look larger and irregular and spread to the fruit.

### Results

Plants were drenched or sprayed with 4 mM solution of phenylalanine or with water and then incubated at high humidity and temperature of 20-25 °C. The plants were naturally infected by a suspension of bacterium cells (10⁷/ml). Phenylalanine and water were applied at 3 days before infection and at the same day of infection. Disease was evaluated according to a 0-100% severity scale where 0=no symptoms (Figures1A-C). Figure 1B - Severity of bacterial speck on tomato plants treated with drench or spray of 4 mM solution phenylalanine. Disease was evaluated on a 0-100% severity of symptoms coverage 13 days after treatment. Bars = Standard Errors; Columns followed by a different letter are significantly different (P≤0.05). Figure 1C - Severity of bacterial speck on tomato plants treated with spray of 4-12 mM solution phenylalanine. Disease was evaluated on a 0-100 % severity of symptoms coverage and calculation of area under disease progress curve (AUDPC) 14 days after treatment. Columns followed by a different letter are significantly different (P≤0.05).

### EXAMPLE 2

### Tuta absoluta

### Background

An insect pest that is a moth belonging to the family Gelechiidae. The larva feeds on tomato plants, producing large galleries in leaves. Tomato is the main host plant, but *T. absoluta* also attacks other crop plants of the including potato, eggplant, pepino, pepper and tobacco and many solanaceous plants. Damage caused by the tomato leaf miner, insect *Tuta absoluta* on tomato leaflet is shown in Figure 3A.

### Results

Tomato plants were grown in pots in a nonheated greenhouse where temperatures were 17-32 °C. The pest spread in the tomato plants naturally. Damage of the pest was evaluated according to a 0-100 % severity scale where 0=no symptoms followed by a calculation of area under disease progress curve (AUDPC) 13 days after treatment. Bars = Standard Errors; Columns followed by a different letter are significantly different (P≤0.05) (Figure 3B).

Severity of damage of the moth *Tuta absoluta* leaf miner on tomato leaves treated with 4 mM drench or spray of Phe solution was evaluated: significantly suppressed by each of the treatments (Fig. 3C). The symptom sizes were separated into small (up to 1 cm long) and large. Both kinds of symptoms were suppressed by the Phe spray and the Phe drench treatments (Fig. 3D). The rate (in %) of larger symptoms was reduced by the spray and by the drench treatments meaning an action of resistance towards the development of leaf miner symptoms after the interaction of the miner with the leaf (Fig. 3E).

Although the invention has been described in conjunction with specific embodiments thereof, it is evident that many alternatives, modifications and variations will be apparent to those skilled in the art.

Citation or identification of any reference in this application shall not be construed as an admission that such reference is available as prior art to the present invention. To the extent that section headings are used, they should not be construed as necessarily limiting.

### EXAMPLE 3

### Tetranychus urticae

*Tetranychus urticae* Koch, the red spider mite (also two-spotted spider mite) is a plant feeding mite of the family Tetranychidae. This mite can feed on tomato, pepper, potato, bean, maize, strawberry and many other crop plants. It sucks the cell contents in leaves causing whitish spots on the leaf surface. Eventually it reduces the photosynthetic ability of the plants, eventually causes leaf mortality and major yield losses.

The red spider mite *Tetranychus urticae* Koch, (also two-spotted spider mite) occurred naturally on the treated tomato plants. The plants were kept in a greenhouse with 18-28°C. Typical scratching symptoms severity were evaluated on each plant; 0 = no infection (all leaves are symptomless) and 100 = leaves were fully covered by symptoms.

### Effect of Phe on red spider mite Tetranychus urticae on tomato plants

Severity of damage of the *Tetranychus urticae* red spider mite on tomato leaves treated with drench or spray of 4 mM Phe solution was significantly suppressed by each of the treatments (Fig. 9).

### EXAMPLE 4

### Bemisia tabaci

Background: *Bemisia tabaci* Gennadius is the silver leaf whitefly (commonly also sweet potato whitefly) is an insect that causes damage to many plant crops by feeding on them and by transmission of virus plant pathogens. It sucks phloem liquid from the leaves, causes whitish dots and secretes honeydew that promotes the development of sooty molds on plant canopies. Many crop plants are affected including tomato, squash, broccoli, cauliflower, cabbage, melon, cotton, carrot, sweet potato, cucumber, pumpkin, and ornamental plants.

### Effect of Phe on silverleaf white fly (Bemisia tabaci) on tomato leaves

The insect silver leaf whitefly *Bemisia tabaci* Gennadius (commonly also sweet potato whitefly) occurred naturally on the treated tomato plants. The plants were kept in a greenhouse with 18-28 °C. Incidence of insect individuals' number on the 10^{th} leaf of the tomato plants was counted.

Incidence of the *Bemisia tabaci* silver leaf whitefly on tomato leaves treated with drench or spray of Phe solution was significantly suppressed by each of the treatments (Fig. 10).

While certain features of the invention have been described herein, many modifications, substitutions, changes, and equivalents will now occur to those of ordinary skill in the art. The scope of the invention is however defined by the appended claims. Subject-matter not falling within their scope is not intended to be part of the invention.

## Claims

1. A method of controlling a pathogenic infection in a plant, comprising contacting said plant with an effective amount of a composition comprising phenylalanine at a concentration of 0.5 to 30 mM, thereby controlling a pathogenic infection in a plant, wherein said pathogenic infection is an arthropod infection, a bacterium infection, or both.

2. The method of claim 1, wherein said arthropod infection is an insect infection, an arachnid infection, or both optionally wherein said insect infection is a moth infection.

3. The method of claim 1, wherein said bacterium infection is a *Pseudomonas* infection.

4. The method of any one of claims 1 to 3, wherein a concentration of said phenylalanine is above 2 mM.

5. The method of any one of claims 1 to 3, wherein said contacting comprises pre-harvest contacting, post-harvest contacting or a combination thereof.

6. The method of any one of claims 1 to 4, wherein said contacting is when said plant is at: a post-blossom stage, a blossom stage, a pre-blossom stage, or any combination thereof.

7. The method of any one of claims 1 to 6, wherein said phenylalanine is formulated in a composition selected from the group consisting of: a dip, a spray, a seed coating, a concentrate, or any combination thereof.

8. The method of any one of claims 1 to 7, wherein said contacting is contacting in the vicinity of or onto: a root, a stem, a trunk, a seed, a fruit, a flower, a leave, or any combination thereof.

9. The method of any one of claims 1 to 8, wherein said contacting is irrigating, drenching, dipping, soaking, injecting, coating, spraying, or any combination thereof.

10. The method of any one of claims 1 to 9, wherein said contacting is repeated at least twice.

11. The method of any one of claims 1 to 10, wherein said contacting is pre-infection, post infection, or a combination thereof, optionally wherein said contacting is in: a storage facility, a greenhouse, an open field, or any combination thereof.

12. The method of claim 2, wherein any one of: (i) said moth is *Tuta absoluta*; and (ii) said arachnid is a spider mite (*Tetranychus urticae*)*.*

13. The method of claim 1, wherein said concentration of said phenylalanine is 4-15 mM.

14. The method of claim 1, wherein said composition is used in combination with one or more other agricultural agents, including pesticides, insecticides, acaricides, fungicides, bactericides, and herbicides.

## Patentansprüche

1. Verfahren zur Bekämpfung einer pathogenen Infektion in einer Pflanze, umfassend das Inkontaktbringen der Pflanze mit einer wirksamen Menge einer Zusammensetzung, die Phenylalanin in einer Konzentration von 0,5 bis 30 mM umfasst, wodurch eine pathogene Infektion in einer Pflanze bekämpft wird, wobei die pathogene Infektion eine Arthropodeninfektion, eine Bakterieninfektion oder beides ist.

2. Verfahren nach Anspruch 1, wobei die Arthropodeninfektion eine Insekteninfektion, eine Spinnentierinfektion oder beides ist, wobei die Insekteninfektion wahlweise eine Motteninfektion ist.

3. Verfahren nach Anspruch 1, wobei die Bakterieninfektion eine *Pseudomonas-*Infektion ist.

4. Verfahren nach einem der Ansprüche 1 bis 3, wobei eine Konzentration des Phenylalanins über 2 mM liegt.

5. Verfahren nach einem der Ansprüche 1 bis 3, wobei das Inkontaktbringen ein Inkontaktbringen vor der Ernte, ein Inkontaktbringen nach der Ernte oder eine Kombination davon umfasst.

6. Verfahren nach einem der Ansprüche 1 bis 4, wobei das Inkontaktbringen erfolgt, wenn sich die Pflanze in einem Nachblütenstadium, einem Blütenstadium, einem Vorblütenstadium oder einer beliebigen Kombination davon befindet.

7. Verfahren nach einem der Ansprüche 1 bis 6, wobei das Phenylalanin in einer Zusammensetzung formuliert ist, die ausgewählt ist aus der Gruppe bestehend aus: einem Dip, einem Spray, einem Samenüberzug, einem Konzentrat oder einer beliebigen Kombination davon.

8. Verfahren nach einem der Ansprüche 1 bis 7, wobei das Inkontaktbringen in der Nähe von oder auf einer Wurzel, einem Stängel, einem Stamm, einem Samen, einer Frucht, einer Blüte, einem Blatt oder einer beliebigen Kombination davon erfolgt.

9. Verfahren nach einem der Ansprüche 1 bis 8, wobei das Inkontaktbringen ein Bewässern, Tränken, Eintauchen, Durchtränken, Injizieren, Beschichten, Besprühen oder eine beliebige Kombination davon ist.

10. Verfahren nach einem der Ansprüche 1 bis 9, wobei das Inkontaktbringen mindestens zweimal wiederholt wird.

11. Verfahren nach einem der Ansprüche 1 bis 10, wobei das Inkontaktbringen vor der Infektion, nach der Infektion oder in einer Kombination davon erfolgt, wobei das Inkontaktbringen wahlweise in einer Lagereinrichtung, einem Gewächshaus, einem offenen Feld oder einer beliebigen Kombination davon erfolgt.

12. Verfahren nach Anspruch 2, wobei eines der folgenden Merkmale zutrifft: (i) die Motte ist *Tuta absoluta*; und (ii) das Spinnentier ist eine Spinnmilbe (*Tetranychus urticae*)*.*

13. Verfahren nach Anspruch 1, wobei die Konzentration des Phenylalanins 4-15 mM beträgt.

14. Verfahren nach Anspruch 1, wobei die Zusammensetzung in Kombination mit einem oder mehreren anderen landwirtschaftlichen Mitteln, einschließlich Pestiziden, Insektiziden, Akariziden, Fungiziden, Bakteriziden und Herbiziden, verwendet wird.

## Revendications

1. Procédé de contrôle d'une infection pathogène dans une plante, comprenant l'étape consistant à mettre en contact ladite plante avec une quantité efficace d'une composition comprenant de la phénylalanine à une concentration allant de 0,5 à 30 mM, contrôlant ainsi une infection pathogène dans une plante,
dans lequel ladite infection pathogène est une infection par les arthropodes, une infection bactérienne ou les deux.

2. Procédé de la revendication 1, dans lequel ladite infection par les arthropodes est une infection par les insectes, une infection par les arachnides, ou les deux éventuellement, dans lequel ladite infection par les insectes est une infection par les mites.

3. Procédé de la revendication 1, dans lequel ladite infection bactérienne est une infection par *Pseudomonas.*

4. Procédé de l'une quelconque des revendications 1 à 3, dans lequel une concentration de ladite phénylalanine est supérieure à 2 mM.

5. Procédé de l'une quelconque des revendications 1 à 3, dans lequel ladite mise en contact comprend une mise en contact pré-récolte, une mise en contact post-récolte ou une combinaison de celles-ci.

6. Procédé de l'une quelconque des revendications 1 à 4, dans lequel ladite mise en contact a lieu lorsque ladite plante est à : un stade post-floraison, un stade de floraison, un stade pré-floraison ou toute combinaison de ceux-ci.

7. Procédé de l'une quelconque des revendications 1 à 6, dans lequel ladite phénylalanine est formulée dans une composition choisie dans le groupe consistant en : un bain, une pulvérisation, un enrobage de semences, un concentré ou toute combinaison de ceux-ci.

8. Procédé de l'une quelconque des revendications 1 à 7, dans lequel ladite mise en contact est une mise en contact à proximité de ou sur : une racine, une tige, un tronc, une graine, un fruit, une fleur, une feuille ou toute combinaison de ceux-ci.

9. Procédé de l'une quelconque des revendications 1 à 8, dans lequel ladite mise en contact est une irrigation, un arrosage, une immersion, un trempage, une injection, un enrobage, une pulvérisation ou toute combinaison de ceux-ci .

10. Procédé de l'une quelconque des revendications 1 à 9, dans lequel ladite mise en contact est répétée au moins deux fois.

11. Procédé de l'une quelconque des revendications 1 à 10, dans lequel ladite mise en contact est une pré-infection, une post-infection, ou une combinaison de celles-ci, éventuellement dans lequel ladite mise en contact est dans : une installation de stockage, une serre, en plein champ, ou toute combinaison de ceux-ci.

12. Procédé de la revendication 2, dans lequel l'un(e) quelconque parmi : (i) ladite mite est *Tuta absoluta*; et (ii) ledit arachnide est un tétranyque (*Tetranychus urticae*)*.*

13. Procédé de la revendication 1, dans lequel ladite concentration de ladite phénylalanine est comprise entre 4 et 15 mM.

14. Procédé de la revendication 1, dans lequel ladite composition est utilisée en combinaison avec un ou plusieurs autres agents agricoles, y compris les pesticides, les insecticides, les acaricides, les fongicides, les bactéricides et les herbicides.
